# EUROPÄISCHE PATENTANMELDUNG

(11) **EP 4 582 015 A1**
(43) Veröffentlichungstag der Anmeldung: **09.07.2025**
(21) Anmeldenummer: 24218350.7
(22) Anmeldetag: 09.12.2024
(51) Int. Cl.: A61B 5/00, A61B 1/07, F21V 8/00, A61C 13/15

(54) **BELEUCHTUNGSSYSTEM MIT EINEM WELLENLÄNGENKONVERTER**

(30) Priorität: 04.01.2024 DE 102024100198
(71) Anmelder: SCHOTT AG, 55122 Mainz (DE)
(72) Erfinder: SCHULTHEIS, Bernd, 55122 Mainz (DE); VIETOR, Jens, 55122 Mainz (DE); SEIDL, Albrecht, 55122 Mainz (DE); MUDERS, Philipp, 55122 Mainz (DE)
(74) Vertreter: Schott Corporate IP

(57) **Zusammenfassung**

Die Erfindung betrifft ein Beleuchtungssystem (1), umfassend ein Handstück (10) mit einer Beleuchtungseinheit (16) und zumindest einem Halbleiter-Leuchtelement (16.1), wobei das zumindest eine Halbleiter-Leuchtelement (16.1) im Betriebszustand eine Abstrahlung (16.3) von im Wesentlichen blauen Lichts im Wellenlängenbereich zwischen 400 nm und 500 nm aufweist, und eine Lichtleiteinheit (20), welche lösbar mit dem Handstück (10) verbunden und/ oder verbindbar ist, wobei die Lichtleiteinheit (20) einen faseroptischen Lichtleitstab (22) mit einer proximalen Endfläche (22.3) und einer distalen Endfläche (22.1) und eine Hülse (21) umfasst, wobei der faseroptische Lichtleitstab (22) in einem Abschnitt ausgehend von der proximalen Endfläche (22.3) in der Hülse (21) mittels eines ersten Klebers (23) fixiert ist, und wobei die Lichtleiteinheit (20) mindestens einen Konverter (24) umfasst, wobei der Konverter (24) die Abstrahlung (16.3) des mindestens einen Halbleiter-Leuchtelements (16.1) im Betriebszustand in eine distale Abstrahlung (22.2) des faseroptischen Lichtleitstabes an dessen distaler Endfläche (22.1) umwandelt, so dass die distale Abstrahlung (22.2) im Betriebszustand im Wesentlichen weißes und/ oder farbneutrales Licht, oder farbiges Licht, zumindest einer Wellenlänge oder zumindest eines Wellenlängenbereiches im sichtbaren Spektralbereich aufweist.

## Beschreibung

### Gebiet der Erfindung

Die Erfindung betrifft ein Beleuchtungssystem, umfassend mindestens ein Handstück mit einer Beleuchtungseinheit und mindestens einem Halbleiter-Leuchtelement, das im Betriebszustand eine Abstrahlung im Wesentlichen blauen Lichtes im Wellenlängenbereich zwischen im Wesentlichen 400 nm und 500 nm aufweist, und einer Lichtleiteinheit, welche lösbar mit dem Handstück verbunden beziehungsweise verbindbar ist, und welche einen faseroptischen Lichtleitstab und eine Hülse umfasst und der faseroptische Lichtleitstab in die Hülse mittels eines ersten Klebers in der Hülse fixiert, mit dieser verklebt beziehungsweise in diese eingeklebt ist und welches im Betriebszustand eine Abstrahlung von im Wesentlichen weißem und/ oder farbneutralem Licht, oder farbigem Licht, zumindest einer Wellenlänge oder zumindest eines Wellenlängenbereiches im sichtbaren Spektralbereich am distalen Ende des Lichtleitstabes, also der Beleuchtungseinheit abgewandt, aufweist.

Derartige Beleuchtungssysteme werden insbesondere zum Aushärten von Zahnfüllungen im Dental-Bereich verwendet. Dabei wird das aus dem Handstück ausgesendete blaue Licht über beispielsweise einen Glasfaserstab zum Zahn geführt und das Zahnfüllmaterial, welches üblicherweise aus einer Mischung von fotoaushärtenden Polymeren und Glas- oder Glaskeramik-Pulver besteht, innerhalb weniger Sekunden ausgehärtet.

Derartige Geräte sind beispielsweise aus den Schriften DE 101 04 579 B4 oder EP 3579786 B1 bekannt.

Die Schrift DE 101 04 579 B4 beschreibt ein Lichthärtegerät, welches eine Halbleiter-Strahlungsquelle umfasst, die in dem Lichthärtegerät montiert ist und Strahlung mindestens teilweise im sichtbaren Spektralbereich ausstrahlt und für das Härten einer im Strahlengang liegenden Masse einschaltbar ist. Die Strahlung weist eine Beleuchtungsstärke von mindestens 200, insbesondere von mindestens 300 mW/cm² auf und die Halbleiter-Strahlungsquelle steht in metallischer und/oder keramischer Wärmeleitverbindung mit einem Basiskörper. Typischerweise wird hierbei eine Peak-Wellenlänge von 440 nm und 470 nm verwendet.

Aus der Schrift EP 3579786 B1 ist eine zahnärztliche Lichtbestrahlungsvorrichtung bekannt, aufweisend eine erste Lichtquelle zum Emittieren von blauem Licht, typischerweise im Bereich von 430 nm bis 480 nm, eine Kamera und mindestens einen ersten Detektor, wobei die Vorrichtung in einem Kameramodus (C), in dem die Kamera aktiviert ist, und in einem Nichtkameramodus (N), in dem die Kamera deaktiviert ist, betreibbar ist, wobei eine Benutzereingabe an dem ersten Detektor in dem Nichtkameramodus (N) den Kameramodus (C) aktiviert, wobei eine Detektion einer Anweisung "Bild einfrieren" der Benutzereingabe über den ersten Detektor in dem Kameramodus (C) ein Einfrieren eines durch die Kamera aufgenommenen Bildes auslöst und eine Detektion einer Anweisung "Kamera aus" der Benutzereingabe über den ersten Detektor bewirkt, dass die Vorrichtung in den Nichtkameramodus (N) umschaltet. Zusätzlich ist in dieser Vorrichtung vorgesehen, dass im Kameramodus (C) statt der blau emittierenden Lichtquelle eine weiß emittierende 2. Lichtquelle dazu geschaltet werden kann. Hierbei handelt es sich um ein komplexes und aufwendig gestaltetes Gerät, welches neben dem Aushärten von Zahnfüllungen auch eine visuelle Kontrolle inkl. Bild-Dokumentation ermöglicht.

Zur Inspektion von Zähnen ist beispielsweise ein Ansatz aus der US 2018256033 A1 bekannt, bei dem ein Aufsatzteil zur Beobachtung von Zahnrissen bereitgestellt wird und das Aufsatzteil einen Spitzenkörper umfasst, welches in ein Kopplungsrohr einer Lichthärtungsvorrichtung eingesetzt ist, von der eine Lichtleiterspitze abgetrennt wurde; ein Führungsrohr zur Rissbeobachtung, das so ausgebildet ist, dass es sich von dem Spitzenkörper um eine vorbestimmte Länge erstreckt, und in den Mund eingesetzt ist. Weiterhin umfasst die Einheit einen Lichtumwandlungsfilter, der mit einem hinteren Ende des Spitzenkörpers gekoppelt ist und blaues Licht in weißes Licht umwandelt, wobei der Lichtumwandlungsfilter durch Beschichtung mit einer roten Farbe und einer grünen Farbe auf beiden Seiten des Spitzenkörpers ausgebildet ist. Nachteilig ist bei diesem Ansatz, dass der Lichtumwandlungsfilter einzeln zwischen Aufsatzteil und der Lichthärtungsvorrichtung eingelegt werden muss, was vergleichsweise aufwendig ist. Zudem geht durch die beiden Farbfilter ein hoher Lichtanteil durch Absorption verloren.

### Aufgabe der Erfindung

Aufgabe der Erfindung ist es daher, ein Beleuchtungssystem bereitzustellen, das für den Praxisbetrieb, also beim zum Beispiel zahnmedizinischen Einsatz, mechanisch robust sowie mit hoher Effizienz weißes oder farbiges Licht bereitstellt und dabei ohne zusätzliche Geräte, insbesondere weitere oder separate Beleuchtungseinrichtungen, Anwendungen wie "Aushärtung" von Füllungen, "Inspektion" beziehungsweise "Kontrolle" von Zähnen, Zahnfleisch oder "Untersuchung" beispielsweise von Gewebe oder dessen Veränderung, insbesondere in der Mundhöhle, ermöglicht, und dabei einfache und sichere Handhabung, kompakte Bauform sowie einen einfachen Wechsel zwischen den verschiedenen Anwendungsfällen ermöglicht beziehungsweise gewährleistet. Weiterhin besteht der Wunsch, möglichst mit vorhandenem Equipment zur Aushärtung von Zahnfüllungen auch die oben zusätzlich benannten Anwendungsfälle mit farbigem und/oder weißem Licht durchzuführen, ohne in zusätzliche, meist teure Spezialgeräte zu investieren.

### Kurzbeschreibung der Erfindung

Die Aufgabe der Erfindung wird bereits durch den Gegenstand der unabhängigen Ansprüche gelöst. Vorteilhafte Ausgestaltungen und Weiterbildungen sind Gegenstand der abhängigen Ansprüche.

Gemäß der Erfindung ist ein Beleuchtungssystem vorgesehen, welches ein Handstück mit einer Beleuchtungseinheit und zumindest einem Halbleiter-Leuchtelement, wobei das zumindest eine Halbleiter-Leuchtelement im Betriebszustand eine Abstrahlung von im Wesentlichen blauen Lichts im Wellenlängenbereich zwischen 400 nm und 500 nm aufweist, und weiter eine Lichtleiteinheit, welche lösbar mit dem Handstück verbunden und/ oder verbindbar ist, umfasst. Die Lichtleiteinheit umfasst dabei einen faseroptischen Lichtleitstab mit einer proximalen Endfläche und einer distalen Endfläche und eine Hülse. Der faseroptische Lichtleitstab ist dabei in einem Abschnitt ausgehend von der proximalen Endfläche, also an dessen Mantelflächen, in der Hülse mittels eines ersten Klebers fixiert. Die Lichtleiteinheit umfasst dabei mindestens einen Konverter, wobei der Konverter die Abstrahlung, also das zum Beispiel blaue Licht, des zumindest einen Halbleiter-Leuchtelements im Betriebszustand in eine distale Abstrahlung des faseroptischen Lichtleitstabes an dessen distaler Endfläche umwandelt, so dass diese distale Abstrahlung im Betriebszustand im Wesentlichen weißes und/ oder farbneutrales Licht, oder farbiges Licht, zumindest einer Wellenlänge oder zumindest eines Wellenlängenbereiches im sichtbaren Spektralbereich aufweist.

Mit anderen Worten, die Lichtleiteinheit weist mindestens einen Konverter auf, der im Betriebszustand die Abstrahlung des Halbleiter-Leuchtelements in Richtung des proximalen Endes des faseroptischen Lichtleitstabes in eine distale Abstrahlung des faseroptischen Lichtleitstabes derart umwandelt, dass diese distale Abstrahlung im Wesentlichen weißes Licht oder farbiges Licht von mindestens einer Wellenlänge oder eines Wellenlängenbereichs des sichtbaren Spektrums elektromagnetischer Strahlung, beispielsweise im roten, grünen, blauen oder gelben Spektralbereich oder einer Mischung von zumindest zwei Farben daraus, entspricht. Solche Konverter werden auch als Wellenlängenkonverter bezeichnet und erlauben also beispielsweise das Licht einer anregenden ersten Wellenlänge (Primärlicht) in Licht insbesondere einer zweiten vom Konverter emittierten Wellenlänge (Sekundärlicht), zum Primärlicht unterschiedlichen Wellenlänge, umzuwandeln. Neben der Konversion von Primärlicht in Sekundärlicht im sichtbaren Spektrum elektromagnetischer Strahlung, ist mittels entsprechender Konverter auch die Konversion in Sekundärlicht beziehungsweise Sekundärstrahlung beispielsweise mindestens einer Wellenlänge oder eines Wellenlängenbereichs des infraroten (IR) oder ultravioletten (UV) Spektrums elektromagnetischer Strahlung möglich.

Je nach Anwendung können auch mehrere, in unterschiedliche Wellenlängen konvertierende Materialien eingesetzt werden und so in mehreren Bereichen des sichtbaren Lichtspektrums Licht bereitgestellt werden, so dass insbesondere auch in deren Mischung unterschiedlich farbiges Licht erhalten werden kann.

Bei entsprechender Lichtmischung durch passend designtes Konvertermaterial vorwiegend hinsichtlich Porosität, Dotierung und Dicke kann so auch weißes Licht generiert werden. Dies ist beispielsweise bei blauen Lichtquellen einer ersten Wellenlänge oder Wellenlängenbereichs in Kombination mit gelbem Licht einer zweiten Wellenlänge oder Wellenlängenbereichs eines Konverters beziehungsweise Konvertermaterials durch entsprechende Mischung des blauen Lichtes, welches den Konverter anregt und teilweise in Konversionslicht umgewandelt wird, und des so erhaltenen konvertierten gelben Lichtes möglich. Damit kann ein für den Praxisbetrieb mechanisch robustes System realisiert werden, dass einerseits mit hoher Effizienz das blaue Licht in weißes oder farbiges Licht umwandelt und andererseits in Bezug auf Handhabung einen einfachen Wechsel zwischen den Anwendungen "Aushärtung" mit insbesondere blauem Licht und "Inspektion", "Kontrolle" oder "Untersuchung" mit beispielsweise weißen oder farbigen Licht ermöglicht, indem die normale, zur Aushärtung verwendete, Lichtleiteinheit ohne Konverter gegen eine Lichtleiteinheit mit Konverter getauscht wird.

Neben blauem Licht im sichtbaren Spektralbereich, wie eingangs beschrieben, ist auch mit UV-Licht mit < 400 nm Wellenlänge eine derartige Konversion bei Einsatz oder Verwendung eines entsprechenden Konvertermaterials grundsätzlich möglich.

Befindet sich der Konverter an der proximalen Endfläche des faseroptischen Lichtleitstabes und überdeckt diese zumindest teilweise, wie dies eine besonders bevorzugte Ausführungsform vorsieht, und befindet sich somit in unmittelbarer Nähe zur Beleuchtungseinheit des Handstücks, wenn die Lichtleiteinheit im Handstück zum Betrieb der Dental-Beleuchtungseinheit eingesteckt ist, so kann mit hoher Effizienz das emittierte blaue Licht, welches damit den Konverter am proximalen Ende des Lichtleitstabes be- oder durchleuchtet, in Weißlicht oder farbiges Licht umgewandelt und an dessen distalem Ende bereitgestellt werden. Zudem kann der Konverter in diesem Fall geschützt durch die Hülse verbaut werden und kommt während der Anwendung nicht mit u.a. Gewebe des Patienten in Kontakt.

In einer vorteilhaften Ausführung des Beleuchtungssystems ist demnach alternativ oder zusätzlich der Konverter der proximalen Endfläche des faseroptischen Lichtleitstabes zugeordnet und überdeckt die proximale Endfläche zumindest teilweise. Im verbundenen Zustand von Lichtleiteinheit und Handstück ist somit der Konverter der Beleuchtungseinheit zugewandt angeordnet.

In bevorzugter Ausführungsform umfaßt oder besteht der Konverter aus einem keramischen Material, wobei im Betriebszustand durch das Konvertermaterial die Abstrahlung der Beleuchtungseinheit in Transmission beeinflussbar ist. Für eine im Wesentlichen weiße beziehungsweise farbneutrale distale Abstrahlung an der distalen Endfläche des faseroptischen Lichtleitstabes umfasst oder besteht der Konverter aus Ce:YAG, für eine farbige Abstrahlung mit Schwerpunkt im roten beziehungsweise rotgelben Spektralbereich des sichtbaren Lichtes umfasst oder besteht der Konverter aus Ce:YAG mit zusätzlicher Gallium (Ga)-Dotierung als Co-Dotierung beziehungsweise für eine farbige Abstrahlung mit Schwerpunkt im grünen Spektralbereich des sichtbaren Lichtes umfasst oder besteht der Konverter aus Ce:LuAG. Durch zusätzliche Dotierung eines Ce:LuAG mit Gadolinium (Gd) kann der Schwerpunkt dessen farbiger Abstrahlung noch weiter in den Grünbereich geschoben werden. Ce:YAG steht hierbei für Cer-dotierten Yttrium-Aluminium-Granat und Ce:LuAG für Cer-dotierten Lutetium-Aluminium-Granat. Alternativ oder zusätzlich kann der Konverter auch aus sogenannten nitridischen oder oxy-nitridischen, meist mit Europium dotierten, keramischen Phosphoren beziehungsweise Leuchtstoffen, wie beispielsweise vom Typ eines Eu:SiN, Eu:SiON und/ oder Eu:SiAlON bestehen oder diese umfassen, die insbesondere Sekundärlicht im grünen und roten Wellenlängenbereich des sichtbaren Spektrums emittieren und ebenfalls durch zusätzliche Elemente, wie beispielsweise Kalzium und/oder Barium, zur Modifikation der Emissionswellenlänge co-dotiert sein können. Durch Einsatz entsprechender Konversionsmaterialien kann auch Konversion von Primärlicht in Sekundärlicht beziehungsweise Sekundärstrahlung in beispielsweise Licht oder Strahlung im infraroten (IR) oder ultraviaoletten (UV) Bereich des Spektrums elektromagnetischer Wellen erreicht werden.

Diese keramischen Materialien als Konverter weisen insbesondere eine hohe Temperaturbeständigkeit insbesondere gegenüber. Kunststoff-basierten Konvertermaterialien auf.

Weiter alternativ kann der Konverter beziehungsweise das Konvertermaterial auch als Materialverbund einer Matrix mit darin eingelagerten Partikeln zumindest eines Leuchtstoffes ausgebildet sein. Somit kann der Konverter auch aus einer Keramikmatrix, Glasmatrix oder einer Polymermatrix beziehungsweise Kunststoffmatrix, zum Beispiel einer Silikonmasse oder Epoxidharz bestehen, in welche ein oder mehrere Leuchtstoffe beziehungsweise sogenannte Phosphore, also fluoreszierende Konversionsstoffe eingelagert sind. Solche Phosphore können angeregt durch Licht einer Wellenlänge oder eines Wellenlängenbereiches (Primärlicht) Licht einer anderen Wellenlänge oder Wellenlängenbereiches (Sekundärlicht) emittieren. Man spricht hier auch von Phosphor-in-Glas, Phosphor-in-Keramik oder Phosphor-in-Silikon. Diese unterscheiden sich deutlich von den keramischen Konverter-Materialien, was sich insbesondere bei den Materialeigenschaften bemerkbar macht, was in nachfolgender Tabelle 1 anhand ausgewählter Eigenschaften dargestellt ist:

**Tabelle 1**

| **Eigenschaft** | **Keramische Konverter** | **Phosphor in Glas** | **Phosphor in Silikon** |
|---|---|---|---|
| **Thermische Leitfähigkeit** | 6 bis 10 W/mK | 0,8 bis 1,2 W/mK | 0,2 bis 1 W/mK |
| **TemperaturBeständigkeit** | > 1000° C / | bis ca. 700° C | bis ca. 160° C |
| | (bis 250° C bei organischer Bindung) | | |
| **Robustheit** | hoch | hoch | niedrig |

Eine hohe thermische Leitfähigkeit ist dabei vorteilhaft, um Überhitzungen der Konverter zu vermeiden oder zumindest deren thermische Belastung zu minimieren, was insbesondere bei hohen Lichtintensitäten, wenn beispielsweise die Konverter mit leistungsstarken Lasern angeregt beziehungsweise beleuchtet werden, relevant ist. Die dabei entstehende Wärme kann dann mit hoher Effizienz zu zum Beispiel zum Konverter korrespondierenden Kühlflächen abgeleitet werden. Damit einhergehend ist auch eine hohe Temperaturbeständigkeit von Interesse, was insbesondere für keramische Konverter und auch für eine Phosphor-dotierte Glasmatrix vorteilhaft ist. Eine Überhitzung des Konvertermaterial kann zu unerwünschten Farbortverschiebungen des emittierten Lichtes und vor allem auch zu verringerter Lichtausbeute durch sogenanntes thermisches Quenching des Konvertermaterials beziehungsweise eines Leuchtstoffes führen.

Die Robustheit bezieht sich hierbei sowohl auf die mechanische Stabilität beziehungsweise Festigkeit als auch insbesondere auf chemische Beständigkeit, die vor allem dann zum Tragen kommt, falls ein Konverter bei der Desinfektion, Sterilisierung oder Wiederaufbereitung, wie diese in der Medizintechnik beziehungsweise medizinischen Anwendungen üblich sind, mit dazu notwendigen Prozessen und/oder Chemikalien beaufschlagt wird oder mit diesen in Kontakt kommt.

Es kann weiterhin vorgesehen sein, dass der Konverter eine Kombination von zumindest zwei Konvertermaterialien umfasst oder daraus besteht. Eine derartige Kombination ist insbesondere bei zuvor beschriebenen Verbundmaterialien als Konvertermaterial, wie Phosphoren in Glas oder in Silikon von Bedeutung.

In einer weiteren vorteilhaften Ausführung des Beleuchtungssystems umfasst oder besteht der Konverter demnach aus zumindest einem der folgenden Materialien: einem keramischen Konvertermaterial vom Typ Ce:YAG, einem keramischen Konvertermaterial vom Typ Ce:YAG mit zusätzlicher Ga-Dotierung, einem keramischen Konvertermaterial vom Typ Ce:LuAG, einem keramischen Konvertermaterial vom Typ Ce:LuAG mit zusätzlicher Gd-Dotierung, einem keramischen Konvertermaterial vom Typ Eu:SiN, Eu:SiON und/ oder Eu:SiAlON, einem Konvertermaterial als Materialverbund einer Glasmatrix mit darin eingelagerten Partikeln zumindest eines Leuchtstoffes, einem Konvertermaterial als Materialverbund einer Keramikmatrix mit darin eingelagerten Partikeln zumindest eines Leuchtstoffes und/oder einem Konvertermaterial als Materialverbund einer Polymermatrix mit darin eingelagerten Partikeln zumindest eines Leuchtstoffes. Der Konverter kann dabei auch eine Kombination von zumindest zwei der vorgenannten Konvertermaterialien umfassen oder daraus bestehen.

Derartige Konverter sind in der Regel eher transluzent bis opak und nur in kleinen Schichtdicken hinreichend transparent, um primäres Licht in Transmission zu sekundärem Licht zu konvertieren beziehungsweise dies zum Beispiel zu Beleuchtungszwecken bereitzustellen. Art des Konverters, zum Beispiel das Material des Konverters beziehungsweise dessen Konversionseigenschaften, und Ausgestaltung, zum Beispiel dessen Dicke, ermöglichen es so die Farbe oder den Farbeindruck des insgesamt bereitgestellten Lichtes einzustellen oder zu steuern. Dies kann auch eine Mischung von Primär- und Sekundärlicht umfassen. Derartige Konverter können sowohl in Transmission beziehungsweise transmissiv oder in Remission beziehungsweise remissiv eingesetzt werden. Transmissiv bedeutet dabei, dass das Primärlicht den Konverter von einer ersten zu einer zweiten Oberfläche durchleuchtet und dabei zumindest teilweise in Sekundärlicht umgewandelt wird, welches dann insbesondere an oder nach der zweiten Oberfläche bereitgestellt wird. In Remission wird eine Oberfläche des Konverters mit Primärlicht beleuchtet, dieses durchdringt diesen zumindest teilweise, wird dabei zumindest teilweise in Sekundärlicht umgewandelt und über Rückstreuung insbesondere an oder nach der beleuchteten Oberfläche bereitgestellt. In beiden Fällen kann je nach Umwandlungsgrad von Primärlicht in Sekundärlicht eine Mischung von beiden oder auch nur Sekundärlicht, bei vollständiger Umwandlung bereitgestellt werden.

Für den transmissiven Einsatz der Konverter, die oft eher transluzent sind, sind Dicken von 100 µm oder geringer, typischerweise 80 µm besonders vorteilhaft. Durch entsprechende Nachbearbeitung, zum Beispiel Schleifen, Läppen oder Politur, sind auch Dicken von 50 µm bis hin zu 30 µm darstellbar, um diese transparenter auszulegen. Je nach Dicke des Konverters und/oder der Art des umfassten Leuchtstoffes wird das in den Konverter eintretende Primärlicht mindestens teilweise in Sekundärlicht umgewandelt, sodass nach Transmission von Primärlicht und Sekundärlicht durch den Konverter Mischlicht aus beiden vorliegt oder bei vollständiger Umwandlung des Primärlichtes auch nur Sekundärlicht. Derartige Konverterelemente lassen sich beispielsweise aus einem Wafer durch mechanisch, abrasive beziehungsweise spanende Verfahren, wie Säge- oder Bohr-Prozesse, besonders vorteilhaft durch Laserbearbeitung in beliebigen Konturen (zum Beispiel quadratisch, rechteckig, vieleckig, polygonal, rund oder unrund) herstellen. Für die Anwendung gemäß der Erfindung sind kreisrunde Scheiben mit einem Durchmesser von 4 mm bis 12 mm bevorzugt welche dem Durchmesser des faseroptischen Lichtstabes, welche in der Regel ebenfalls rund hergestellt vorliegen, im Wesentlichen entspricht.

Der Konverter kann auch aus mehreren Konverterelementen auch aus unterschiedlichen Konvertermaterialien mit gegebenenfalls je unterschiedlichen Dicken der Konverterelemente zusammengesetzt beziehungsweise zusammengestellt werden. So können zum einen mehrere Konverterelemente aus dünnen Plättchen gestapelt angeordnet werden und/ oder zum anderen auch aus Segmenten dargestellt werden, also beispielsweise ein runder Konverter aus mehreren Kreissegmenten, die je auch aus unterschiedlichen Konvertermaterialien bestehen können, zusammengesetzt werden. Wie zuvor beschrieben überdeckt der Konverter die proximale Endfläche des Lichtleitstabes zumindest teilweise, kann diese aber auch vollständig bedecken oder überdecken. Eine nur teilweise Abdeckung der proximalen Endfläche des Lichtleitstabes durch den Konverter kann erwünscht oder bevorzugt sein, wenn eine bestimmte, gegebenenfalls vorgegebene Mischung primären und sekundären Lichtes erreicht werden soll. In einem solchen Fall, aber auch grundsätzlich, findet im Lichtleitstab zumindest teilweise eine Mischung beziehungsweise Homogenisierung hin zu dessen distalen Ende des in diesen proximal eingekoppelten Lichtes statt.

Besonders vorteilhaft ist es demnach, wenn der Konverter des Beleuchtungssystems alternativ oder zusätzlich als ein oder mehrere dünne, scheibenförmige Konverterelemente zumindest eines Konvertermaterials ausgebildet ist, welche die proximale Endfläche des faseroptischen Lichtleitstabes zumindest teilweise abdecken und welche jeweils eine Dicke von 30 µm bis 200 µm, bevorzugt von 50 µm bis 150 µm, besonders bevorzugt von 80 µm bis 100 µm aufweisen.

Zusätzliche Maßnahmen, wie beispielsweise die Veränderung der Querschnittsfläche des Lichtleitstabes durch Verjüngungen oder Aufweitungen können zudem die distale Abstrahlcharakteristik, zum Beispiel deren Abstrahlwinkel, beeinflussen beziehungsweise ermöglichen deren Einstellung und können so auch die proximale Einkopplung in den Lichtleitstab begünstigen.

In vorteilhafter Ausgestaltung des Beleuchtungssystems ist alternativ oder zusätzlich vorgesehen, dass das eine oder die mehreren Konverterelemente des Konverters mittels eines zweiten Klebers auf die proximale Endfläche des faseroptischen Lichtleitstabes und/ oder untereinander, insbesondere blasenfrei, verklebt sind und dieser zweite Kleber im Wesentlichen optisch klar, transparent und dauerelastisch im ausgehärteten beziehungsweise verarbeiteten oder vernetzten Zustand ist.

Unter optisch klar, transparent im Sinne der Erfindung wird hier verstanden, dass der zweite Kleber im Wesentlichen ohne Eigenfarbe ist und nicht streuend im Wellenlängenbereichs des den Kleber durchtretenden Lichtes wirkt und keine, zumindest keine wesentliche Abschwächung des diesen durchtretenden Lichtes bewirkt, also beispielsweise zumindest eine Transmission von 90% bei einer Dicke von 1 mm für diese Wellenlängen beziehungsweise in diesem Wellenlängenbereich aufweist. Eine blasenfreie Verklebung ist insbesondere dann von Bedeutung, wenn die Beleuchtungseinheit zumindest eine Lichtquelle aufweist, die aufgrund deren Leistung lokal oder flächig derart Wärme abstrahlt, beziehungsweise in den Konverter am proximalen Ende einträgt, dass sich etwaige vorhandene Blasen zu stark ausdehnen können und es so zu Schädigungen, zu Delamination bis hin zur Zerstörung der Verklebung führen kann.

Zudem kann vorgesehen sein, dass dieser zweite Kleber hinsichtlich seines Brechungsindexes an den des faseroptischen Lichtleitstabs angepasst ist beziehungsweise sich nur wenig, beispielsweise Δn ≤ 0,1, von diesem unterscheidet. Dies reduziert Reflektionsverluste an den jeweils zugehörigen Grenzflächen.

Hinsichtlich der Verarbeitung kann auch vorteilhaft sein, wenn dieser zweite Kleber beim Applizieren eine geringe Viskosität beziehungsweise selbst-nivellierende Fließeigenschaften aufweist und UV-Licht und/ oder heiß-vernetzend verarbeitbar beziehungsweise aushärtbar ist. Dieser zweite Kleber unterscheidet sich damit gegebenenfalls von dem ersten Kleber, mit dem der faseroptische Lichtleitstab in die Hülse der Lichtleiteinheit verklebt ist. Der erste Kleber kann, aber muss nicht, derartige optische Anforderungen erfüllen. Im Gegenteil kann es für den ersten Kleber vorteilhaft sein diesen auch transluzent oder opak und/oder eingefärbt, beispielsweise schwarz eingefärbt vorzusehen, um etwaiges Streulicht aus oder um die Hülse zu vermeiden oder zumindest zu reduzieren.

Bei einer Konvertierung in beziehungsweise Bereitstellung von farbigem und/ oder von im Wesentlichen Weiß-Licht als distale Abstrahlung ist es von Vorteil, dass der Farbort bei erwünschter farbiger beziehungsweise die Farbtemperatur bei weißer und/ oder farbneutraler distaler Abstrahlung durch die Zusammensetzung des Konverters, durch die Dicke des Konverters oder der Konverterelemente und Porosität des KonverterMaterials anforderungsgemäß und/oder gezielt eingestellt werden kann beziehungsweise einstellbar ist. Beispielsweise verschiebt sich der Farbort der distalen Abstrahlung, insbesondere wenn bei blauer Anregungswellenlänge gelb emittierende Ce:YAG basierte keramische Konverter eingesetzt werden, zu kaltweißem Licht beziehungsweise Weißlicht mit hoher Farbtemperatur (> 5000 K als Kaltweiß), je dünner der Konverter ausgeführt ist, da in diesem Fall der durch den Konverter durchdringende Blaulichtanteil höher ist. Mit einem dickeren Konverter kann hier aus gleichem Konvertermaterial eher neutrales- oder gar warm-weißes Licht beziehungsweise Weißlicht mit niedriger Farbtemperatur (typ. im Bereich von 4000 K bis 5000 K als Neutralweiß; typ. < 3000 K als Warmweiß) erhalten werden.

Weitere Einstellmöglichkeiten ergeben sich durch eine materialseitige Anpassungen des Konvertermaterials, zum Beispiel durch Erhöhung oder Erniedrigung der Dotierungen, also zum Beispiel im Falle eines Ce:YAG-Leuchtstoffes beziehungsweise keramischen Ce:YAG Konvertermaterials dessen Gehalt an Ce und/ oder gegebenenfalls weiterer Komponenten oder Co-Dotierungen. Dies, wie zuvor beschrieben beispielsweise als eine Ga Co-dotierung. Somit sind also die Konversionseigenschaften durch Veränderung der Zusammensetzung des Konvertermaterials einstellbar. Ebenfalls kann die Konvertierung in beziehungsweise Bereitstellung von farbigem und/ oder von im Wesentlichen Weiß-Licht als distale Abstrahlung durch die Porosität des Konverters beziehungsweise des Konvertermaterials beeinflusst werden.

Im Falle von Verbundmaterialien als Konverter spielt hinsichtlich der Zusammensetzung des Konvertermaterials auch der Volumenanteil an als Partikel oder Pulver in der zugehörigen Matrix eingebrachten Leuchtstoffe eine Rolle. Diese wernde häufig in Korngrößen von unterhalb einem µm, wenigen µm bis einige 10µm bereitgestellt. Je größer der Volumenanteil an Leuchtstoffen hierbei ist, umso mehr oder stärker wird Primärlicht in Sekundärlicht umgewandelt. Die Partikel des Leuchtstoffes beziehungsweise auch die Grenzflächen von Partikeln und Matrix wirken in diesen Verbundmaterialien außerdem als Streuelemente, insbesondere für das Sekundärlicht, aber auch gegebenenfalls nicht umgewandeltes Primärlicht.

Ebenso können bei den vorgenannten Konvertern beziehungsweise deren Materialien, also nicht nur bei Verbundmaterialien, sondern auch den keramischen Konvertern, deren Streuung durch deren Porosität beeinflusst werden. So lassen sich mittels der Porosität sowohl der Weg des anregenden Primärlichtes als auch der Weg des emittierten Sekundärlichtes modifizieren. Somit lassen sich also mittels der Porosität der Konvertermaterialien beziehungsweise der Konverterelemente, wobei insbesondere auch die Grenzfläche zwischen Poren und diese umgebendem Material streuend wirkt, die Abstrahlung des Konverters, also der Farbort bei erwünschter distaler Abstrahlung insgesamt farbigen Lichtes beziehungsweise die Farbtemperatur bei erwünschter im Wesentlichen weißer Abstrahlung variieren beziehungsweise einstellen. Wird also beispielsweise ein keramischer Konverter beziehungsweise Konverterelement, welches Ce-dotierten YAG bestimmter Zusammensetzung umfasst oder daraus besteht, von einer Seite mit blauem Anregungslicht beleuchtet oder durchstrahlt, so emittiert dieser gelbes Licht, welches bei passend eingestellter Dicke und bestimmter Porosität des Konverters auf der durchstrahlten Seite zum Beispiel neutral weißes Licht ergibt. Erhöht man nun die Porosität, so verschiebt sich die Farbtemperatur in Richtung wärmeres Weiß und im umgekehrten Falle einer Erniedrigung Porosität hin zu kälterem Weiß. Bei konstanter Porosität dieses Konverters kann ebenso über die Variation der Dicke die Farbtemperatur weißen Lichtes variiert werden, d.h. mit reduzierter Dicke in Richtung kälteren Weiß beziehungsweise erhöhter Dicke hin zu wärmerem Weiß.

Demgemäß ist in vorteilhaften Ausführungen des Beleuchtungssystems vorgesehen, dass der Farbort bei farbiger distaler Abstrahlung oder die Farbtemperatur bei weißer beziehungsweise farbneutraler distaler Abstrahlung durch die Zusammensetzung des Konverters und/ oder des einen oder der mehreren Konverterelemente, durch die Dicke des Konverters und/ oder des einen oder der mehreren Konverterelemente, und/oder durch die Porosität des Konverters und/oder des einen oder der mehreren Konverterelemente einstellbar ist beziehungsweise diese können so eingestellt werden.

Es sind also insbesondere im Weißbereich je nach Ausgestaltung des Konverters sowohl kaltweißes Licht (Farbtemperatur ca. > 5000 K), neutralweißes Licht (Farbtemperatur ca. zwischen 4000 K und 5000 K) beziehungsweise warmweißes Licht (Farbtemperatur ca. < 3000 K) oder auch farbneutrales Licht darstellbar. Dabei wird unter farbneutralem Licht eben solches Licht verstanden, welches sich im L-a-b Farbraum im Wesentlichen entlang der L-Achse befindet beziehungsweise mit geringer Farbabweichung Delta E von insbesondere < 2, vorzugsweise < 1, bei gegebenem L-Wert um die L-Achse liegt.

In einer weiteren vorteilhaften Ausführungsform des Beleuchtungssystems ist alternativ oder zusätzlich vorgesehen, dass auf dem Konverter beziehungsweise auf dem außenliegenden Konverterelement eine Abdeckung angebracht ist, derart dass der Konverter beziehungsweise das außenliegende Konverterelement zumindest vollständig abgedeckt oder überdeckt ist, wobei diese Abdeckung eine Glasscheibe, eine Kunststoffscheibe, eine Saphirscheibe oder eine Quarzglasscheibe umfasst oder daraus besteht und eine Dicke im Bereich von 30 µm bis 500 µm, vorzugsweise von 50 µm bis 200 µm aufweist, und wobei diese Abdeckung ebenfalls mit dem zweiten Kleber mit dem Konverter verbunden ist. Das außenliegende Konverterelement ist dabei, falls der Konverter aus mehreren Konverterelementen gegebenenfalls unterschiedlicher Konvertermaterialien aufgebaut ist, das Konverterelement, welches als letztes von zumindest zweien auf das proximale Ende des Lichtleitstabes beziehungsweise mit dem größten Abstand dazu angeordnet beziehungsweise aufgebracht wurde.

Diese scheibenförmige Abdeckung beziehungsweise Abdeckscheibe dient dabei dem Schutz des Konverters, insbesondere wenn dieser oder zumindest eines der Konverterelemente vergleichsweise porös eingestellt ist. Damit kann insbesondere bei den im Medizinbereich üblichen Aufbereitungsmethoden wie beispielsweise das Autoklavieren (typ. bis zu 140° C, > 3 bar Wasserdampf und bis zu wenige bis einige 10 Minuten Einwirkzeit pro Zyklus, Anzahl der Zyklen >> 100) ein chemischer und mechanischer Schutz des Konverters realisiert werden. Die Materialien der Abdeckung sind entsprechend chemisch resistent auszulegen, sowie optisch derart, dass im Betriebszustand die Einstrahlung des Lichtes der Beleuchtungseinheit im Wesentlichen ungehindert, ohne Streuung durch die Abdeckung möglich ist. Die Abdeckung beziehungsweise deren Material ist also klar, transparent, d.h. wie zuvor schon bzgl. des zweiten Klebers beschrieben im Wesentlichen nicht streuend und farblos oder ohne Eigenfarbe, insbesondere im relevanten Wellenlängenbereich, auszulegen. Die Abdeckung weist also beispielsweise zumindest in diesem Wellenlängenbereich eine Transmission von mehr als 80% bei einer Dicke von 1 mm auf. Als bevorzugte Abdeckung haben sich beispielsweise dünne Plättchen aus Borosilikat-Glas erwiesen. Die Abdeckung schließt demnach, gegebenenfalls in der umgebenden Hülse, das proximale Ende des Lichtleitstabes, auf dem der Konverter angeordnet ist, ab und die Abdeckung ist somit im in das Handstück eingebauten Zustand der im Handstück befindlichen Beleuchtungseinheit mit dessen Lichtquelle zugewandt angeordnet. Insgesamt erhöht die Anbindung einer Abdeckung an den Konverter die eingangs beschriebene Robustheit und Praxistauglichkeit des Systems weiter.

Dabei ist für die Abdeckscheibe alternativ oder zusätzlich vorteilhaft vorgesehen, dass deren thermischer Ausdehnungskoeffizient an den des Konvertermaterial angepasst ist, wobei die Differenz der thermischen Ausdehnungskoeffizienten kleiner 6,0 ppm/k, bevorzugt kleiner 4,5 ppm/K, meist bevorzugt kleiner 3,5 ppm/K ist. Idealerweise unterscheiden sich die thermischen Ausdehnungskoeffizienten von Abdeckscheibe und Konvertermaterial um nicht mehr als 1ppm/K und sind bevorzugt nicht größer als 10 ppm/K. Typischerweise weisen Keramische Konverter des Typs Ce:YAG beispielsweise einen thermischen Ausdehnungskoeffizient um 6,5 ppm/K, solche der nitridischen Leuchtstoffe, wie z.B. eines Eu:SiN, Eu:SiON und/ oder Eu:SiAlON Typs, einen thermischen Ausdehnungskoeffizient um 3 ppm/K auf. Für die Abdeckscheibe geeignete Gläser umfassen oder bestehen aus z.B. Borosilikatgläsern, wie D263^{®} mit einem thermischen Ausdehnungskoeffizienten um 7.2 ppm/K, Borofloat33^{®}, Mempax^{®} oder AF32^{®} mit einem thermischen Ausdehnungskoeffizienten um 3.2 bis 3.3 ppm/K der SCHOTT AG. Ebenfalls geeignet sind Gläser die einen thermischen Ausdehnungskoeffizienten um 8 ppm/k bis um 9,5 ppm/K aufweisen, wie beispielsweise Kalk-Natron-Gläser oder B270^{®}, Xensation^{®}, AS87^{®} der Anmelderin. Etwaige verbleibende Differenzen der thermischen Ausdehnungskoeffizienten können durch den zweiten Kleber an- beziehungsweise ausgeglichen werden. Eine derartige Anpassung der thermischen Ausdehnungskoeffizienten begünstigt die Robustheit und Lebensdauer des System und verhindert mögliche Schädigungen aufgrund thermomechanischer Belastung, wie diese zum Beispiel bei der oben genannten Aufbereitung auftreten können, zumindest aber werden derartige mögliche Schädigungen verzögert.

Zur Vermeidung von Reflektionsverlusten ist in einer weiteren vorteilhaften Ausführungsform des Beleuchtungssystems alternativ oder zusätzlich vorgesehen, dass der Konverter und/oder die Abdeckung zumindest auf der der Beleuchtungseinheit zugewandten Seite eine reflexionsmindernde Beschichtung aufweist. Derartige Schichten sind beispielsweise sogenannte λ/4-Schichten (zum Beispiel bei 450 nm Lichtwellenlänge ca. 115 bis 120 nm dick) oder bestehen beispielsweise aus einer Abfolge von mehreren Schichten aus SiO₂ im Wechsel mit TaO₂.

In einer weiteren vorteilhaften Ausgestaltung des Beleuchtungssystems kann alternativ oder zusätzlich vorgesehen sein, dass zwischen dem Konverter und der proximalen Endfläche des faseroptischen Lichtleitstab ein Farbfilter angeordnet ist. Derartige Farbfilter können zur Feinanpassung beziehungsweise anforderungsgerechten Einstellung der distalen Abstrahlung des faseroptischen Lichtleitstabes, beispielsweise von Farbwerten und/ oder Farborten insbesondere bei farbiger distalen Abstrahlung beziehungsweise von Farbtemperatur insbesondere bei im Wesentlichen weißer oder farbneutraler distaler Abstrahlung, vorgesehen werden. Dieser Farbfilter kann zum Beispiel als Filterglas-Plättchen zwischen dem Konverter und dem proximalen Ende des faseroptischen Lichtleitstabes und/ oder als Schichtsystem auf dem faseroptischen Lichtleitstab oder auf dem Konverter, an dessen dem faseroptischen Lichtleitstabes zugewandten Seite, vorgesehen sein. So kann aus dem konvertiertem Licht beziehungsweise aus dem nach dem Konverter in Richtung der proximalen Endfläche des Lichtleitstabes bereitgestellten Licht ein bestimmter Wellenlängenbereich herausgefiltert werden. Natürlich lässt sich ein derartiger Farbfilter grundsätzlich auch auf die distale Endfläche des faseroptischen Lichtleitstabes vorsehen, allerdings wäre dies im Hinblick auf die Robustheit des Systems weniger bevorzugt. Bevorzugt ist ein solcher Farbfilter ebenfalls mit dem zweiten Kleber an der entsprechend vorgesehenen Position fixiert.

In einer weiter vorteilhaften Ausführungsform des Beleuchtungssystems ist alternativ oder zusätzlich vorgesehen, dass die Hülse im Bereich des Konverters und/ oder der Abdeckung eine Aufweitung des Innendurchmessers aufweist, so dass ein Kleber-Reservoire ausgebildet wird oder ausbildbar ist. Dies erleichtert, vereinfacht beziehungsweise verbessert den Zusammenbau und erlaubt die Vermeidung von Kleberüberschuß, welcher sonst auf dem Konverter beziehungsweise auf der Abdeckung verbleiben kann und die optisch wichtigen Flächen beeinträchtigen würde. Volumen-Toleranzen beim Dosieren des zweiten Klebers können also damit auch aufgefangen werden, so dass ein Übertritt von Kleber auf die optischen relevanten Flächen des Konverters oder der Abdeckung vermieden werden kann.

Bevorzugte Verwendungen des Beleuchtungssystems sind insbesondere solche zur visuellen Kontrolle von Zahnfüllungen und/ oder des Zahnfleischs, zur Sichtbarmachung von Belägen oder Gewebeveränderungen, insbesondere in der Mundhöhle, sowie zur visuellen Kontrolle von Klebeprozessen und/ oder Kleberaushärtung in medizinischen oder industriellen Anwendungen. Die visuelle Kontrolle von Zahnfüllungen und / oder zur Zahnfleisch-Kontrolle sieht im Wesentlichen weißes Licht vor, wobei zur Sichtbarmachung von Belägen oder Gewebeveränderungen auch farbiges Licht zur Anwendung kommt. Allgemein können mit einem derartigen Beleuchtungssystem also auch visuelle Gewebe-Untersuchungen im Mund und Rachenraum, zum Beispiel am Zahnfleisch, an der Zunge und an Zahnoberflächen unter unterschiedlichen Beleuchtungsarten durchgeführt werden. Dies wird auch durch den einfachen Wechsel der Lichtleiteinheit ermöglicht, welche je nach notwendiger oder angeforderter Beleuchtungsart mit weißem oder farbigen Licht in das Handstück mit der Beleuchtungseinheit eingesetzt beziehungsweise ausgetauscht werden kann, wobei die Lichtleiteinheiten jeweils mit entsprechenden Konvertern ausgestattet sind, gegebenenfalls auch ohne Konverter vorgesehen sein können.

Mit einem erfindungsgemäßen Beleuchtungssystem können auch weitere medizinische oder industrielle Verklebeprozesse oder Untersuchungen durchgeführt oder bedient werden, bei denen zum Beispiel mit blauem Licht zunächst eine Kleberhärtung erfolgt und anschließend eine visuelle Kontrolle mit weißem oder farbigen Licht nach erfolgter Kleberaushärtung durchgeführt werden soll oder muss. Beispiele, aber nicht begrenzt hierauf, aus dem Medizinbereich sind gefäßchirurgische Verklebeprozesse von zum Beispiel Haut oder Knochen. Im industriellen Umfeld können dies Verklebeprozesse im Bereich Elektronik sein, bei denen nach erfolgter Verklebung beispielsweise Kontakt-verklebungen einer visuellen Inspektion unterzogen werden müssen.

### Figurenbeschreibung

Die Erfindung wird nachfolgend anhand von Figuren näher beschrieben.

Es zeigen
Fig. 1 einen schematischen Aufbau eines Beleuchtungssystems bestehend aus Handstück und Lichtleiteinheit,
Fig. 2 eine Ausschnittvergrößerung im Bereich des proximalen Endes der Lichtleiteinheit und
Fig. 3 ein CIE-Farbraumdiagramm zur Farbort-Veränderung durch den Konverter.

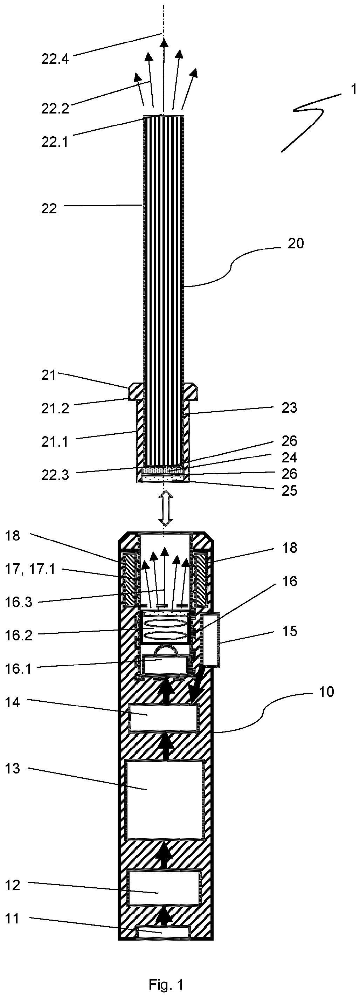

Figur 1 zeigt schematisch in einer Schnittdarstellung den Aufbau eines Beleuchtungssystems 1 gemäß der Erfindung.

Das Beleuchtungssystem 1 besteht aus einem Handstück 10 und einer damit lösbaren verbundenen oder verbindbaren beziehungsweise steckbaren Lichtleiteinheit 20, wobei das Handstück 10 üblicherweise ein Lade-Interface 11 zur Aufladung einer entsprechender Energie-Speichereinheiten 13 aufweist und die Aufladung mit einer Lade-Kontrolleinheit 12 überwacht beziehungsweise der Ladezustand optisch angezeigt werden kann. Die Energie-Speichereinheit 13 wird meist durch wiederaufladbare Batterien, also Akkumulatoren beziehungsweise Sekundärbatterien gebildet. Alternativ sind auch herkömmliche Batterien, also Primärbatterien, denkbar, die aber für den Praxisbetrieb weniger geeignet sind. Auch denkbar ist eine kabelgebundene Energieversorgung. Über eine Beleuchtungs-Kontrolleinheit 14, welche über mindestens ein Bedienelement 15 verfügt, kann eine Beleuchtungseinheit 16 angesteuert werden. Dabei kann üblicherweise der Startzeitpunkt, die Lichtintensität und die Zeitdauer der Beleuchtung eingestellt beziehungsweise vom Anwender vorgegeben werden.

Die Beleuchtungseinheit 16 besteht üblicherweise aus einem oder mehreren Halbeiter-Leuchtelementen 16.1 in Form von einer oder mehreren LEDs oder Laserdioden, oder Kombinationen aus beiden. Zudem ist häufig noch ein optisches Element 16.2 vorgesehen, welches das abgestrahlte Licht des Halbleiter-Leuchtelementes 16.1 bündelt beziehungsweise kollimiert, so dass idealerweise das meiste oder der überwiegende Teil des Lichtes, das im Betriebszustand von dem einen oder mehreren Halbeiter-Leuchtelementen 16.1 abgestrahlt wird, in die Lichtleiteinheit 20 eingekoppelt werden kann und somit die Intensität des bereitgestellten Lichtes insbesondere am distalen Ende der Lichtleiteinheit gesteigert wird und damit zum Beispiel die Behandlungsdauer, wie beispielsweise bei der Aushärtung von lichthärtenden Zahnfüllungen, verkürzt werden kann. Das optische Element 16.2 besteht üblicherweise aus einer Linse oder einem Linsen-System und kann zusätzlich eine planare Abdeckscheibe als Schutz umfassen.

Da diese Handstücke 10 zur Aushärtung von im blauen- bis nahen UV-Bereich lichthärtenden oder lichtvernetzenden Zahnfüllungen verwendet werden, ist im Betriebszustand die Abstrahlung 16.3 der Beleuchtungseinheit 16 im blauen Spektralbereich des sichtbaren Lichtes zwischen 400 nm und 500 nm angesiedelt. Je nach Aushärtemechanismen der verwendeten Zahnfüllmaterialien beziehungsweise deren vernetzbaren Harzsystemen sind insbesondere die Wellenlängen 405 nm und 450 nm von Interesse, um eine schnelle und vollständige Aushärtung beziehungsweise Vernetzung der Zahnfüllung zu erreichen. Gebräuchlich sind aber auch andere Wellenlängen, wie sie eingangs beschrieben wurden. Oft werden daher auch farblich unterschiedlich abstrahlende Halbleiter-Leuchtelemente 16.1 in einer Beleuchtungseinheit 16 verwendet, um eine optimale kurze Aushärtezeit insgesamt und aber auch vollständige Durchhärtung zu erzielen.

Das Handstück 10 weist zudem einen Aufnahmeabschnitt 17 zur Aufnahme der Lichtleiteinheit 20 auf, wobei eine hinsichtlich der Geometrie genaue Passung 17.1 vorgesehen ist, welche mit der Passung 21.1 einer Hülse 21 der Lichtleiteinheit 20 korrespondiert. Entscheidend für die Auslegung der Passungen 17.1 und 21.1 ist hier trotz Lösbarkeit beiden Baugruppen ein fester, sicherer Sitz dieser Komponenten in beziehungsweise aneinander zu gewährleisten. Dies unterstützend oder alternativ, aber in Fig. 1 nicht dargestellt, können noch Dichtungen in Form von beispielsweise O-Ringen vorgesehen sein, die zur weiteren Fixierung aber auch zur Abdichtung dienen. In modernen Handstücken 10 können sich auch im Bereich des Aufnahmeabschnittes 17 Magnete 18 befinden, die mit magnetisierbaren Inlays (beispielsweise aus magnetisierbarem Edelstahl) in der Hülse 21 der Lichtleiteinheit 20 kräftemäßig korrespondieren und so ebenfalls für einen festen Halt der Lichtleiteinheit 20 im Handstück 10 während des Betriebs sorgen.

Das Kernstück der Lichtleiteinheit 20 bildet ein faseroptischer Lichtleitstab 22, der üblicherweise mittels eines ersten Klebers 23 passgenau in die Hülse 21 eingeklebt ist. Dazu werden üblicherweise transluzente oder opake, insbesondere schwarz opake Kleber auf Epoxyd- oder Silikonbasis verwendet. Die Hülse 21, welche üblicherweise aus Edelstahl oder aus einem temperaturbeständigen Kunststoff (zum Beispiel Polyphenylsulfon PPSU oder Polyphenylensulfid PPS, diese ggf. auch mit Glasfaser-Verstärkung) besteht, weist neben einem Schaft, welcher zum Aufnahmeabschnitt 17 des Handstückes 10 korrespondiert, noch einen Anschlag 21.2 auf, der die Einstecktiefe ins Handstück 10 definiert.

Derartige faseroptische Lichtleitstäbe 22 geeignete Materialien und Verfahren zu deren Herstellung sind beispielsweise aus der DE 10 2013 208 838 B4 beziehungsweise DE 10 2004 034 603 B4 der Anmelderin bekannt und können, wie darin gezeigt, gerade gestreckt ausgeführt sein. Diese können aber gegebenenfalls auch insbesondere an deren distalem Ende ggü. der Längsachse 22.4 des Lichtleitstabes 22 zumindest abschnittsweise abgewinkelt und auch zumindest abschnittsweise zusätzlich verjüngt ausgeführt sein.

Die zuvor beschrieben Merkmale beschreiben typ. Dental-Aushärteeinheiten, welche bereits seit vielen Jahren zur Aushärtung von Zahnfüllungen zum Einsatz kommen.

Gemäß der Erfindung umfasst die Lichtleiteinheit 22 mindestens einen Konverter 24, der im Betriebszustand die Abstrahlung 16.3 des Halbleiter-Leuchtelements 16.1 in eine distale Abstrahlung 22.2 des faseroptischen Lichtleitstabes 22 derart umwandelt, dass die distale Abstrahlung 22.2 im Wesentlichen weißem Licht oder farbigem Licht im beispielsweise grünen, gelben oder roten Spektralbereich entspricht. Der Konverter 24 wird dabei in Transmission betrieben, also durchleuchtet, wobei das blaue Licht des Halbleiter-Leuchtelementes 16.1 durch den Konverter 24 durchtritt und zumindest teilweise, bei beispielsweise Verwendung eines Konvertermaterials eines gelb emittierenden Ce:YAG-Leuchtstoffes, in gelbes Licht umgewandelt wird, so dass daraus in Summe weißes Licht oder anders farbiges Licht emittiert wird.

Veränderungen der distalen Abstrahlung 22.2, beispielsweise des Farbbildes, also bei farbigen Licht des Farbortes oder bei weißem Licht der Farbtemperatur, welche zum Beispiel durch Alterung der anregenden Lichtquelle, konkret des Halbleiter-Leuchtelementes 16.1 eintritt, kann durch Anpassung und/oder Nachregeln der Leistung des Halbleiter-Leuchtelementes 16.1 kompensiert werden.

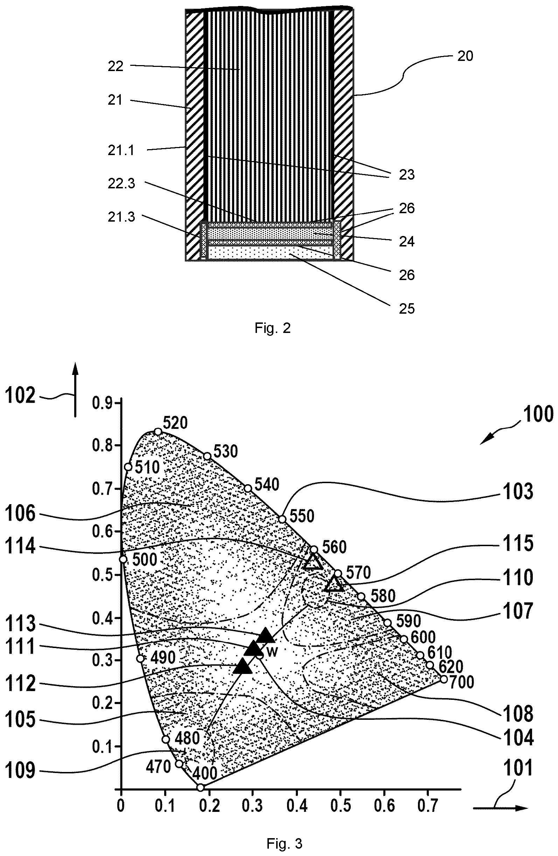

Weiter ist in bevorzugter Ausführung vorgesehen, dass der Konverter 24 direkt auf die proximale Endfläche 22.3 des faseroptischen Lichtleitstabes 22 stoffschlüssig aufgeklebt ist. Wie bereits eingangs erwähnt, kann der Konverter 24 ein plättchenförmiges Konverterelement sein, wie dies beispielhaft in der Fig. 1 als auch als Ausschnittsvergrößerung in Fig. 2 zu sehen ist, oder aber auch als eine Kombination von unterschiedlichen Konverterelementen aus gegebenenfalls unterschiedlichen Konvertermaterialien als Konverter 24 ausgeführt sein. In einer weiter bevorzugten Variante schließt der Konverter mit der Hülse 21 bündig ab, so dass Überstände vermieden werden. Dabei ist vorgesehen, dass der oder die Konverter-Scheiben mittels eines zweiten Klebers 26 mit der proximalen Endfläche 22.3 des faseroptischen Lichtleitstabes 22 beziehungsweise auch untereinander blasenfrei verklebt sind. Dieser zweite Kleber 26 ist idealerweise klar transparent aushärtbar und besitzt selbstnivellierende Eigenschaften. Grundsätzlich geeignet sind UV-härtende und/ oder unter Temperatur aushärtende Klebersysteme auf Acrylat- oder Epoxid-Basis. Darüber hinaus kommen auch Flüssig-Silikone (Liquid Silicon Rubber, LSR) in Betracht, welche unter Hitze vernetzen. Diese zeichnen sich durch gute Dauerelastizität aus und können auch die materialbedingten Unterschiede beim thermischen Ausdehnungs-koeffizienten zwischen faseroptischen Lichtleitstab 22 und Konverter 24 ausgleichen.

Optional kann zusätzlich auf dem Konverter 24, wie dies die Fig. 1 und 2 zeigen, noch eine Abdeckung 25 beziehungsweise Scheibe aufgeklebt sein, welche den Konverter 24 vollständig abdeckt, wobei diese Abdeckung 25 idealerweise aus einem chemisch resistenten Glas (z.B. aus Borosilikat-Glas), aus Saphir oder aus Quarzglas besteht und eine Dicke im Bereich von 30 µm bis 500 µm, vorzugsweise von 50 µm bis 200 µm aufweist, wobei diese Abdeckung 25 ebenfalls mit dem zweiten Kleber 26 mit dem Konverter 24 verklebt ist.

Als weitere Option kann vorgesehen sein, dass diese Abdeckung 25 zumindest auf der der Beleuchtungseinheit 16 zugewandten Seite eine reflexionsmindernde Beschichtung aufweist. Derartige Beschichtungen sind auch als λ/4-Schichten bekannt und werden oft als Schichtfolge appliziert.

Idealerweise weist die Hülse 21 im Bereich des Konverters 24 und/ oder der Abdeckung 25 eine Aufweitung des Innendurchmessers auf, so dass ein definiertes Kleber-Reservoire 21.3 ausgebildet beziehungsweise ausbildbar ist. Dies vereinfacht den Klebeprozeß und hilft Ausschuß infolge überschüssigem Kleber auf dem Konverter 24 beziehungsweise auf der Abdeckung 25 zu vermeiden.

Das Prinzip der Lichtkonversion lässt sich dabei mit einem CIE-Farbraumdiagramm 100, wie in Fig. 3 dargestellt, erläutern.

Fig. 3 zeigt schematisch im Einzelnen ein CIE-Farbraumdiagramm 100, wie es aus der Fachliteratur zu entnehmen ist, mit den Farbachsen cₓ 101 und c_{y} 102 und dem Weißbeziehungsweise Unbunt-Punkt W 104, welcher durch die Koordinaten cₓ = 0,33 und c_{y} = 0,33 definiert ist. Am Rande des Farbraums ist die Wellenlänge 103 in nm für das sichtbare Licht angegeben, beginnend mit 400 nm im Blau-Bereich 105, über den Grün-Bereich 106 und den Gelb-Bereich 107 bis 700 nm im Rot-Bereich 108 dargestellt.

Wird nun blaues Licht zwischen typischerweise 400 nm und 500 nm, üblicherweise 405 nm und/ oder 450 nm, emittiert, was dem Emissionsbereich 109 des Halbleiter-Leuchtelementes 16.1 entspricht, kann durch eine zumindest teilweise Umwandlung von blauem Licht in gelbes Licht, bei Verwendung beispielsweise eines im Wesentlichen gelb emittierenden Ce-dotierten YAG Leuchtstoffes beziehungsweise einem keramischen Konverter 24 daraus, was der konvertierten Emission 110 entspricht, entlang der Verbindungslinie zwischen dem Emissionsbereich 109 und der konvertierten Emission 110 farbiges und auch weißes Licht erzeugt werden.

Durch Art und/ oder Zusammensetzung des Konverters 24, Porosität des Konverters 24, prozentualem Anteils der Dotierstoffe im Konverter 24 und/ oder Dicke des Konverters 24 können zwischen der blauen Anregung und der Emission im gelben Bereich verschiedene Farborte beziehungsweise Farbtemperaturen im Weißbereich um den Weiß- beziehungsweise Unbunt-Punkt W 104 erzeugt werden. So kann mit höher porösem Konvertermaterial oder dickerem Material eher farbiges oder wärmer weißes Licht (Warm-Weiß 113) beziehungsweise mit dünneren Konverter-Dicken oder geringerer Porosität ein eher Kalt-Weiß 112 erzeugt werden. Ein Konvertermaterial, welches weniger porös ist beziehungsweise die Dicke etwas größer ist ergibt demnach ein Kalt-Weiß 112. Bei geeigneter Balance zwischen der den Konverter 24 durchdringenden blauen Anregungsstrahlung und dem Anteil an konvertiertem Licht, kann auch ein Neutral-Weiß 111 in der Nähe des Weiß- beziehungsweise Unbunt-Punkt W 104 erzeugt werden.

Für eine Konversion von blauem Licht in Weißlicht kommen insbesondere keramische Konvertermaterialien in Frage welche Ce:YAG (Yttrium-Aluminium-Granat, welches mit Cer dotiert ist) enthalten oder aus diesen Material bestehen. Zusätzlich können noch Zumischungen von Gallium (Ga) enthalten sein, um die Emission, also die Wellenlänge oder den Wellenlängenbereich des emittierten Sekundärlichtes, zu beeinflussen.

Abhängig beziehungsweise im Zusammenspiel von Zusammensetzung des Konverters 24, dessen Dicke, dessen Porosität und/oder Anteils an Leuchtstoff, insbesondere bei Verbundmaterialien als Konverter 24, wie zum Beispiel Phosphor in Glas oder in Silikon, ist beispielsweise auch farbiges Licht als distale Abstrahlung 22.2 erzielbar.

So lässt sich beispielsweise eine grünliche Gesamtemission 114 (siehe Fig. 3) durch Ce:LuAG erzielen, einem Lutetium-Aluminium-Granat, welcher mit Cer dotiert ist. Eine ins Rötliche geschobene gelbe Gesamtemission 115 (siehe Fig. 3) lässt sich dagegen mit einem Konverter 24 aus Ce:YAG, einem Yttrium-Aluminium-Granat, welcher mit Cer dotiert ist und Zumischungen von Gallium (Ga) enthält, erzielen. Eine ins Grünliche geschobene grüne Gesamtemission lässt sich mit einem Konverter 24 aus Ce:LuAG, einem Lutetium-Aluminium-Granat, welcher mit Cer dotiert ist und Zumischungen von Gadolinium (Gd) enthält, erzielen.

Der oder die Konverter 24 beziehungsweise Konverterelemente umfassen oder bestehen bevorzugt aus einem keramischen Material und sind beispielsweise in den Schriften WO 13060731 A2 oder WO 13139619 A1 der Anmelderin beschrieben.

Ausführungsbeispiele (AB) für solche keramischen Konverter-Materialien sind:

| Bezeichnung | Eigenschaften |
|---|---|
| AB1 | gelb emittierender Konverter 24 vom Typ Ce:YAG |
| AB2 | gelb emittierender Konverter 24 vom Typ Ce:YAG mit erhöhter Ce-Dotierung |
| AB3 | ins Rötliche geschobener gelb emittierender Konverter 24 vom Typ Ce:YAG mit Ga-Dotierung |
| AB4 | ins Grünliche geschobener Konverter 24 vom Typ Ce:LuAG |
| AB5 | weiter in den Grün-Bereich geschobener grün emittierender Konverter 24 vom Typ Ce:LuAG mit Gd-Dotierung |

Die Schichtdicke der Konverter 24 beträgt typischerweise 80 µm bis 150 µm, bevorzugt 80 µm bis 100 µm.

Für den eingangs beschriebenen Anwendungsfall haben sich folgende Konverter-Varianten als vorteilhaft erwiesen, wobei diese bevorzugt polierte Oberflächen aufweisen und eine anti-reflex Beschichtung aufweisen.

So lassen sich mit einem Konverter 24 nach den obigen Ausführungsbeispielen AB1 und AB2 in einem erfindungsgemäßen Beleuchtungssystem bei einer Dicke von ca. 80µm eine weiße distale Abstrahlung erhalten. Bei größeren Dicken eine distale Abstrahlung, welche mit zunehmender Dicke gelber wird.

Um ins Rötliche oder Grünliche verschobene distale Abstrahlungen zu erhalten können Konverter 24 nach den AB3 bis AB5 mit Dicken von 80 µm eingesetzt werden. Auch hier wird die distale Abstrahlung mit zunehmender Dicke roter beziehungsweise grüner.

### Bezugszeichen

- 1: Beleuchtungssystem

- 10: Handstück
- 11: Lade-Interface
- 12: Lade-Kontrolleinheit
- 13: Energie-Speichereinheit
- 14: Beleuchtungs-Kontrolleinheit
- 15: Bedienelement
- 16: Beleuchtungseinheit
- 16.1: Halbleiter-Leuchtelement
- 16.2: Optisches Element
- 16.3: Abstrahlung
- 17: Aufnahmeabschnitt
- 17.1: Passung
- 18: Magnete

- 20: Lichtleiteinheit
- 21: Hülse
- 21.1: Passung
- 21.2: Anschlag
- 21.3: Kleber-Reservoire
- 22: faseroptischer Lichtleitstab
- 22.1: distale Endfläche
- 22.2: distale Abstrahlung
- 22.3: proximale Endfläche
- 22.4: Längsachse
- 23: Erster Kleber
- 24: Konverter
- 25: Abdeckung
- 26: Zweiter Kleber
- 100: CIE-Farbraumdiagramm
- 101: Farbachse cₓ
- 102: Farbachse c_{y}
- 103: Wellenlänge
- 104: Weiß-/Unbunt-Punkt W
- 105: Blau-Bereich
- 106: Grün-Bereich
- 107: Gelb-Bereich
- 108: Rot-Bereich
- 109: Emissionsbereich
- 110: konvertierte Emission
- 111: Neutral-Weiß
- 112: Kalt-Weiß
- 113: Warm-Weiß
- 114: grünliche Gesamtemission
- 115: rot/gelbliche Gesamtemission

## Patentansprüche

1. Beleuchtungssystem (1), umfassend
ein Handstück (10) mit einer Beleuchtungseinheit (16) und zumindest einem Halbleiter-Leuchtelement (16.1), wobei
das zumindest eine Halbleiter-Leuchtelement (16.1) im Betriebszustand eine Abstrahlung (16.3) von im Wesentlichen blauen Lichts im Wellenlängenbereich zwischen 400 nm und 500 nm aufweist,
und eine Lichtleiteinheit (20), welche lösbar mit dem Handstück (10) verbunden und/ oder verbindbar ist, wobei
die Lichtleiteinheit (20) einen faseroptischen Lichtleitstab (22) mit einer proximalen Endfläche (22.3) und einer distalen Endfläche (22.1) und eine Hülse (21) umfasst, wobei
der faseroptische Lichtleitstab (22) in einem Abschnitt ausgehend von der proximalen Endfläche (22.3) in der Hülse (21) mittels eines ersten Klebers (23) fixiert ist, und wobei
die Lichtleiteinheit (20) mindestens einen Konverter (24) umfasst, wobei der Konverter (24) die Abstrahlung (16.3) des mindestens einen Halbleiter-Leuchtelements (16.1) im Betriebszustand in eine distale Abstrahlung (22.2) des faseroptischen Lichtleitstabes an dessen distaler Endfläche (22.1) umwandelt, so dass die distale Abstrahlung (22.2) im Betriebszustand im Wesentlichen weißes und/ oder farbneutrales Licht, oder farbiges Licht, zumindest einer Wellenlänge oder zumindest eines Wellenlängenbereiches im sichtbaren Spektralbereich aufweist.

2. Beleuchtungssystem (1) nach Anspruch 1, wobei der Konverter (24) der proximalen Endfläche (22.3) des faseroptischen Lichtleitstabes (22) zugeordnet ist und die proximale Endfläche (22.3) zumindest teilweise überdeckt und im verbundenen Zustand von Lichtleiteinheit (20) und Handstück (10) der Beleuchtungseinheit (16) zugewandt angeordnet ist.

3. Beleuchtungssystem (1) nach Anspruch 1 oder 2, **gekennzeichnet durch** zumindest eines der folgenden Merkmale
- der Konverter (24) umfaßt ein oder besteht aus einem keramischen Konvertermaterial vom Typ Ce:YAG,
- der Konverter (24) umfaßt ein oder besteht aus einem keramischen Konvertermaterial vom Typ Ce:YAG mit zusätzlicher Ga-Dotierung,
- der Konverter (24) umfaßt ein oder besteht aus einem keramischen Konvertermaterial vom Typ Ce:LuAG,
- der Konverter (24) umfasst ein oder besteht aus einem keramischen Konvertermaterial vom Typ Ce:LuAG mit zusätzlicher Gd-Dotierung,
- der Konverter (24) umfaßt ein oder besteht aus einem keramischen Konvertermaterial vom Typ Eu:SiN, Eu:SiON und/ oder Eu:SiAlON,
- der Konverter (24) umfaßt ein oder besteht aus einem Konvertermaterial als Materialverbund einer Glasmatrix mit darin eingelagerten Partikeln zumindest eines Leuchtstoffes,
- der Konverter (24) umfaßt ein oder besteht aus einem Konvertermaterial als Materialverbund einer Keramikmatrix mit darin eingelagerten Partikeln zumindest eines Leuchtstoffes,
- der Konverter (24) umfaßt ein oder besteht aus einem Konvertermaterial als Materialverbund einer Polymermatrix mit darin eingelagerten Partikeln zumindest eines Leuchtstoffes,
- der Konverter (24) umfasst oder besteht aus einer Kombination von zumindest zwei Konvertermaterialien.

4. Beleuchtungssystem (1) nach einem der vorhergehenden Ansprüche, wobei der Konverter (24) als ein oder mehrere scheibenförmige Konverterelemente zumindest eines Konvertermaterials ausgebildet ist, welche jeweils eine Dicke von 30 µm bis 200 µm, bevorzugt von 50 µm bis 150 µm, besonders bevorzugt von 80 µm bis 100 µm aufweisen.

5. Beleuchtungssystem (1) nach Anspruch 4, wobei das eine oder die mehreren Konverterlemente des Konverters (24) mittels eines zweiten Klebers (26) auf die proximale Endfläche (22.3) des faseroptischen Lichtleitstabes (22) und/ oder untereinander verklebt sind, wobei der zweite Kleber (26) im Wesentlichen optisch klar, transparent und dauerelastisch im ausgehärteten Zustand ist.

6. Beleuchtungssystem (1) nach einem der vorhergehenden Ansprüche, **gekennzeichnet durch** zumindest eines der folgenden Merkmale
- der Farbort bei farbiger distaler Abstrahlung (22.2) oder die Farbtemperatur bei weißer beziehungsweise farbneutraler distaler Abstrahlung (22.2) ist durch die Zusammensetzung des Konverters (24) und/ oder des einen oder der mehreren Konverterelemente einstellbar,
- der Farbort bei farbiger distaler Abstrahlung (22.2) oder die Farbtemperatur bei weißer beziehungsweise farbneutraler distaler Abstrahlung (22.2) ist durch die Dicke des Konverters (24) und/ oder des einen oder der mehreren Konverterelemente einstellbar,
- der Farbort bei farbiger distaler Abstrahlung (22.2) oder die Farbtemperatur bei weißer beziehungsweise farbneutraler distaler Abstrahlung (22.2) ist durch die Porosität des Konverters (24) und/oder des einen oder der mehreren Konverterelemente einstellbar.

7. Beleuchtungssystem (1) nach einem der vorhergehenden Ansprüche, wobei auf dem Konverter (24) oder auf dem außenliegenden Konverterelement eine Abdeckung (25) angebracht ist, derart dass der Konverter (24) beziehungsweise das außenliegende Konverterelement zumindest vollständig abgedeckt oder überdeckt ist, wobei
diese Abdeckung (25) eine Glasscheibe, eine Kunststoffscheibe, eine Saphirscheibe oder eine Quarzglasscheibe umfasst oder daraus besteht und eine Dicke im Bereich von 30 µm bis 500 µm, vorzugsweise von 50 µm bis 200 µm aufweist, und wobei diese Abdeckung (25) ebenfalls mit dem zweiten Kleber (26) mit dem Konverter (24) verbunden ist.

8. Beleuchtungssystem (1) nach Anspruch 7, wobei
der Konverter (24) und/ oder die Abdeckung (25) zumindest auf der der Beleuchtungseinheit (16) zugewandten Seite eine reflexionsmindernde Beschichtung aufweist.

9. Beleuchtungssystem (1) nach einem der vorhergehenden Ansprüche, wobei zwischen dem Konverter (24) und der proximalen Endfläche (22.3) des faseroptischen Lichtleitstabes (22) ein Farbfilter angeordnet ist.

10. Beleuchtungssystem (1) nach einem der vorhergehenden Ansprüche, wobei die Hülse (21) im Bereich des Konverters (24) und/ oder der Abdeckung (25) eine Aufweitung des Innendurchmessers aufweist, so dass ein Kleber-Reservoire (21.3) ausgebildet wird oder ausbildbar ist.

11. Verwendung des Beleuchtungssystems (1) nach einem der vorhergehenden Ansprüche zur visuellen Kontrolle von Zahnfüllungen und/ oder des Zahnfleischs, zur Sichtbarmachung von Belägen oder Gewebeveränderungen sowie zur visuellen Kontrolle von Klebeprozessen und/ oder Kleberaushärtung in medizinischen oder industriellen Anwendungen.
